# EUROPEAN PATENT SPECIFICATION

(11) **EP 4 655 033 B1**
(45) Date of publication and mention of the grant of the patent: **09.12.2026**
(21) Application number: 24703478.8
(22) Date of filing: 24.01.2024
(51) Int. Cl.: A61M 16/04

(54) **INNER CANNULA FOR TRACHEOSTOMY TUBE**
INNENKANÜLE FÜR TRACHEOSTOMIETUBUS
CANULE INTERNE POUR TUBE DE TRACHÉOTOMIE

(30) Priority: 24.01.2023 DE 102023101606
(43) Date of publication of application: 03.12.2025
(73) Proprietor: Coloplast A/S, 3050 Humlebaek (DK)
(72) Inventor: HAHN, Andreas, 55291 Saulheim (DE); SCHNELL, Ralf, 63500 Seligenstadt (DE)
(86) International application number: PCT/DK2024/050011
(87) International publication number: WO 2024/156322

(56) References cited:
- WO-A1-2014/132016
- US-A- 6 024 730

## Description

The present invention relates to an inner cannula for insertion into an outer cannula or outer tube of a tracheostomy tube, wherein the inner cannula comprises a tubular cannula wall which is composed of flexible plastics material and which extends in a longitudinal direction between a proximal end of the inner cannula and an opposite distal end of the inner cannula, wherein the inner cannula comprises, in the longitudinal direction thereof, a round arch portion with an inner arcuate side and an outer arcuate side. In addition, the present invention relates to a tracheostomy tube with an outer cannula and an inner cannula of the aforementioned type arranged in the outer cannula, to a method for producing such a tracheostomy tube and to the use of the inner cannula of the aforementioned type for the exchange of a used inner cannula arranged in the outer cannula of a tracheostomy tube.

A tracheostomy tube (also tracheostomy cannula) enables the long-term ventilation of patients who have had an entry to the trachea (tracheostoma) created through the soft tissues of the neck for example by means of a surgical procedure (tracheotomy). In the case of spontaneously breathing tracheostomized patients, tracheostomy tubes can also serve to keep the stoma open. In addition, tracheostomy tubes can also be used as a connection possibility for accessories, such as HMEs or tracheostoma valves. For the optimal transition between the tracheostoma and the trachea, tracheostomy tubes typically comprise a round arch portion.

For their intended use, many tracheostomy tubes contain an inner cannula (also inner tube) which is arranged in an outer cannula (also outer tube) and which is in many cases also able to be locked therein, whereas other tracheostomy tubes are used without an inner cannula being arranged therein. There are also tracheostomy tubes which can be used both with and without an inner cannula. In the prior art, the term "tracheostomy tube" is used such that it encompasses all these variants without explicitly revealing whether the tracheostomy tube does or does not contain an inner cannula.

Tracheostomy tubes with an inner cannula have the advantage that the inner cannula can be changed in a very uncomplicated manner, when it is blocked by, for example, mucus, whilst the outer cannula can remain on the patient. Furthermore, there is the possibility of quickly and easily changing between different types of inner cannulas in order to adapt the function of the tube to requirements that may have changed. It is for example possible for inner cannulas with phonation windows to be inserted into a windowed outer cannula if the patient wishes to speak. By contrast, if the patient has to be ventilated, a closed inner cannula without a window can again be used. A change of the inner cannula can be carried out much more easily and more gently for the patient than a complete change of the outer cannula. This is particularly the case with freshly created stomas, since it is specifically here that dangerous complications can frequently occur when changing tracheostomy tubes.

There are an enormous variety of differently embodied tracheostomy tubes on the market, with which attempts are made to meet the various requirements. In view of the sometimes very different anatomies and physical conditions, tracheostomy tubes differ, among other things, in terms of their length, in terms of the inner and outer diameter of the cannula, in terms of the position, the radius and the bending angle of the round arch portion and in terms of the softness of the materials used.

In spite of the multiplicity of available tracheostomy tubes, the physician using them is often unable to predict with 100 percent certainty which tube shape and geometry is optimal for the respective patient. This is made more difficult by the fact that the requirements may also change if the patient moves.

Tracheostomy tubes with variably bendable cannulas are therefore proposed in the prior art. However, the bendability must not come at the cost of the kink resistance, which can be tested in accordance with DIN Standard ISO 5366:2017 (kinking test). If such tracheostomy tubes can be equipped with inner cannulas, the inner cannulas must also meet the corresponding requirements with respect to the kink resistance. In order to achieve the desired flexibility with simultaneous kink resistance, use is for example made of tracheostomy tubes with spiral-reinforced cannula tubes, wherein the spiral reinforcement can consist of metal or plastic and is typically integrated into the cannula wall.

In this respect, metal-reinforced tracheostomy tubes are not suitable for every patient, since these are not compatible for technical reasons with possibly necessary MRI diagnostics. Tracheostomy tubes with spiral reinforcement are usually obtained by extrusion, thermoforming and/or dip coating methods, all of which are methods associated with relatively large manufacturing tolerances in comparison with injection moulding, which is problematic in terms of design particularly in the case of tracheostomy tubes with phonation windows and inner cannulas. In addition, spiral-reinforced cannulas can be manufactured only with relatively large wall thicknesses in the range of typically 1.7 to 2 mm, which can problematically be at the cost of the cannula cross section available for breathing. Owing to the lower stiffness of plastic compared with the metals that are otherwise typically used, such as stainless steel or nitinol, a spiral reinforcement composed of plastic usually results in even higher wall thicknesses. This is presumably also a reason why spiral-reinforced inner cannulas have hitherto not prevailed on the market.

EP 3 116 573 A1 describes a method in which an extruded hose piece is structured by means of a blow moulding process in such a way that a pattern of two intersecting diagonal grooves is produced. However, the inner surface of the cannula is also structured by the blow moulding method, with the result that secretions can cling there more easily, which is critical from the point of view of hygiene.

WO 2004/101048 A3 describes a soft tracheostomy tube with an inner cannula composed of PTFE. However, PTFE is relatively expensive, and problematic hydrofluoric acid vapours can arise during production and disposal.

WO 2015/118288 A1 describes an inner cannula comprising a very thin cannula wall which is borne on its outer side by a structural frame consisting of a multiplicity of ribs arranged on a longitudinally central strip.

The inner cannula described in DE 10 2007 011 930 B3 also consists of a thin plastics layer which is stabilized by a support structure consisting in this case of a hose-like braid of threads and/or fibres. In both cases, the improvement in the kink resistance is achieved merely by way of a relatively expensive and at least two-stage production process.

WO 2014/132016 A1 discloses a tracheostomy tube including an inner cannula having a smooth bore and outer surface both circular in section. The bore is offset from the centre of the inner cannula towards the inside of the bend of the outer tube,

US 6,024,730 A discloses an inner cannula for a tracheostomy tube assembly formed from a tubular member having two parallel rows of slots formed along its length.

In order to provide an inner cannula for a tracheostomy tube that does not suffer from the disadvantages mentioned above, the invention proposes an inner cannula as claimed in claim 1.

The present invention is defined by the appended claims. Subject-matter which does not fall under the scope of the claims is not part of the invention.

The inner cannula proposed according to the invention can be readily produced with a corresponding tool by the injection moulding method, which can be carried out technically without great expenditure, and the desired flexibility is provided by the lower wall thickness on the outer arcuate side, whilst the required minimum kink resistance is also ensured by the greater wall thickness on the inner arcuate side.

In the context of the present invention, the terms "distal" and "proximal" are used from the perspective of a physician using the tracheostomy tube, i.e. the proximal end is that end of the ventilation device which remains outside the patient's body after the insertion into the trachea (machine end), whilst the distal end is inserted into the patient's trachea (patient end).

In connection with the present invention, the term "round arch portion" denotes the curved portion of the inner cannula. In the embodiments in which the inner cannula is curved over the entire length, the round arch portion correspondingly encompasses the entire length of the inner cannula.

The terms "outer arcuate side" and "inner arcuate side" denote the external portion and, respectively, the internal portion of the round arch, wherein the transition from the outer arcuate side to the inner arcuate side in a circumferential direction lies halfway between the outermost point of the outer arcuate side and the innermost point of the inner arcuate side.

In connection with the present invention, the expression "average wall thickness" denotes the wall thickness which can be computationally ascertained from the area of a cross section through the cannula wall along a cannula longitudinal portion running on the inner arcuate side or a cannula longitudinal portion running on the outer arcuate side. If the considered longitudinal portion measures for example 100 mm in the longitudinal direction of the cannula and the area of the cross section extends through the cannula wall in this longitudinal portion over 250 mm², the average wall thickness of the considered longitudinal portion is 2.5 mm.

In some embodiments, the considered longitudinal portion of the outer arcuate side is only the outer arcuate portion which runs on the outer arcuate side through the apex of the round arch and which extends linearly from proximal to distal over at least 30%, at least 50%, at least 75%, at least 90% or over the entire length of the outer arcuate side in the longitudinal direction of the inner cannula.

In some embodiments, the considered longitudinal portion of the inner arcuate side is only the inner arcuate portion which runs on the inner arcuate side through the apex of the round arch and which extends linearly from proximal to distal over at least 30%, at least 50%, at least 75%, at least 90% or over the entire length of the inner arcuate side in the longitudinal direction of the inner cannula.

In some embodiments, the considered longitudinal portion of the outer arcuate side can also comprise, in addition to the longitudinal portion running around the outermost circumference on the outer arcuate side or around the innermost circumference of the inner arcuate side, longitudinal portions running parallel thereto. If the round arch portion of the inner cannula is considered in cross section relative to the longitudinal axis thereof, the average wall thickness which is lower according to the invention is present on the outer arcuate side compared to the inner arcuate side for each longitudinal portion which extends in a region in the longitudinal direction that extends over 10% of the total outer circumference of the channel wall cross section in the circumferential direction of the cannula around the longitudinal portion running around the outermost circumference on the outer arcuate side and/or for each longitudinal portion which extends in a region in the longitudinal direction that extends over 10% of the total outer circumference of the cannula wall cross section in the circumferential direction of the cannula around the longitudinal portion running around the innermost circumference on the inner arcuate side.

A greater wall thickness on the inner arcuate side may be achieved in that, at least in the region of the longitudinal portion running on the inner arcuate side and of the longitudinal portion running on the outer arcuate side, the inner circumference and the outer circumference of the cannula wall are each circular, elliptical or oval in cross section perpendicular to the central longitudinal axis of the inner cannula and are selected, and arranged, such that the average wall thickness of the longitudinal portion on the inner arcuate side is at least 40% greater than the average wall thickness of the longitudinal portion on the outer arcuate side.

By way of example, in the embodiments in which the outer circumference and the inner circumference of the cannula wall are each circular, the circle centre of the circular cross section of the inner circumference, proceeding from a concentric arrangement with the circle centre of the circular cross section of the outer circumference, is arranged offset in the direction of the outermost point of the outer arcuate side of the outer circumference. In this way, the wall thickness of the cannula wall increases continuously from the outermost point of the outer arcuate side to the innermost point of the inner arcuate side, as a result of which the average wall thickness on the inner arcuate side is greater than the average wall thickness on the outer arcuate side.

A greater wall thickness on the inner arcuate side is also achieved in that the cannula wall comprises indentations in the cannula wall in the region of the longitudinal portion running on the outer arcuate side, by means of which indentations the average wall thickness of the cannula wall is reduced in comparison to the opposite longitudinal portion running on the inner arcuate side. Irrespective of whether they are provided on the inner arcuate side or the outer arcuate side, such indentations may be arranged both on the outer side of the cannula wall and on the inner side of the cannula wall. They are preferably arranged only on the outer side of the cannula wall.

In this case, the size and shape of the indentations on the inner arcuate side and/or the size and shape of the indentations on the outer arcuate side are each selected such that the average wall thickness of the longitudinal portion on the inner arcuate side is at least 40% greater than the average wall thickness of the longitudinal portion on the outer arcuate side.

Due to the different wall thickness, the optimal ratio of flexibility to kink resistance is achieved already at relatively low wall thicknesses. In certain embodiments, the average wall thickness of the cannula wall lies in the range of 0.3 to 1.7 mm, preferably in the range of 0.3 to 1.5 mm. If the average wall thickness in the round arch portion on the outer arcuate side is for example 0.30 mm in such embodiments, the average wall thickness on the inner arcuate side has to be 0.42 mm or more. If the average wall thickness on the outer arcuate side is 1.0 mm, the average wall thickness on the inner arcuate side has to be at least 1.4 mm.

According to the invention, the optimal ratio of flexibility to kink resistance is achieved in that the average wall thickness on the inner arcuate side is at least 40% greater than the average wall thickness on the outer arcuate side. In certain embodiments, it may be desired, depending on the individual requirement profile, for the inner arcuate side to have an even more considerable reinforcement compared with the outer arcuate side. In some embodiments, the average wall thickness on the inner arcuate side is therefore at least 50% or even at least 60% greater.

The optimal ratio of flexibility to kink resistance is additionally also finely adjusted in that, in certain embodiments, indentations are provided in the cannula wall at least in the round arch portion and result in a lower wall thickness in these regions. In the region of the indentations, the wall thickness may, for example, lie in the range of 0.15 to 0.4 mm, whilst the wall thickness of the cannula wall otherwise lies in the range of 0.3 to 1.5 mm.

The shape of the indentations in the cannula wall is quite variable, provided that the requirements in terms of the different average wall thicknesses are met. In certain embodiments, the indentations in the cannula wall extend in a ring-like, clasp-like or spiral-like manner over the cannula wall in the circumferential direction. The indentations can be designed so as to either be continuous or interrupted in certain portions. This enables an even more extensive optimization of the ratio of flexibility to kink resistance and maximum freedom with regard to the individual adaptation to the particular needs of the respective patient.

The shape of the indentations in the cannula wall may also be designed differently in different portions and regions for the optimization of the ratio of flexibility to kink resistance. By way of example, in certain embodiments, the shape of the indentations on the outer arcuate side differs from the shape of the indentations on the inner arcuate side.

The maximum depth of the indentations on the inner arcuate side is greater than the maximum width of the indentations. By way of example, in some embodiments, the maximum depth of the indentations on the inner arcuate side is 10 to 70% greater than the maximum width of the indentations. In particular embodiments, the maximum depth of the indentations on the inner arcuate side lies in the range of 0.5 to 1.2 mm and/or the maximum width of the indentations on the inner arcuate side lies in the range of 0.3 to 8 mm.

Due to the fact that the depth of the indentations is greater than the width thereof, the kink resistance is increased significantly, since the upper ends of the flanks of the indentations abut against one another from a certain degree of bending and are thus supported against one another. The indentations on the outer arcuate side are characterized in that the maximum depth of the indentations on the outer arcuate side is lower than the maximum width of the indentations. In certain embodiments, the maximum depth of the indentations on the outer arcuate side is 10 to 70% lower than the maximum width of the indentations. In specific embodiments, the maximum depth of the indentations on the outer arcuate side is 0.3 to 0.5 mm and/or the maximum width of the indentations on the outer arcuate side is 0.5 to 1.5 mm.

With indentations on the outer arcuate side which are less deep than they are wide, particularly good flexibility of the outer arcuate side is ensured, which is advantageous particularly when introducing the tracheostomy tube into the trachea and simultaneously contributes to a relatively high wear comfort.

In a specific embodiment, the indentations run in a ring-like manner over the entire circumference of the cannula wall, wherein the individual rings are designed such that, on the inner arcuate side, the maximum depth of the indentations is greater than the maximum width thereof, and, on the outer arcuate side, the maximum depth of the indentations is lower than the maximum width thereof.

In an alternative specific embodiment, the indentations run in a ring-like manner over the entire circumference of the cannula wall, wherein, between two of these rings, the plastics material of the cannula wall is embodied as a sealing ring, the circular outer circumference of which is 5 to 15% greater than the circular circumference of the inner side of the cannula wall of the outer cannula in which the inner cannula is intended to be arranged. The thickness of the sealing ring is preferably < 0.5 mm so that the sealing ring has the necessary flexibility to yield slightly, and sealingly lie against the inner wall of the outer cannula, when the inner cannula is inserted into an outer cannula of a tracheostomy tube.

The above-described embodiment enables ideal sealing between the inner cannula and the outer cannula already when a single sealing ring is provided. However, in certain embodiments, it may be advantageous if two or three such sealing rings are provided either directly alongside one another, that is to say separated only by a respective interposed indentation, or at a spacing of multiple indentations.

The region in which the at least one sealing ring of the above-described type is provided may be in the round arch portion, in the region between the round arch portion and the distal end or in the region between the round arch portion and the proximal end. Preferably, the region in which at least one sealing ring of the above-described type is provided is in the region between the round arch portion and the distal end of the inner cannula. In such embodiments, the inner circumference of the outer cannula is particularly preferably tapered in the direction of the distal end.

Since the centre of the round arch of a cannula may be particularly susceptible to kinking, in certain embodiments reinforcing elements are provided at least at or around the apex of the round arch at the outer circumference of the cannula wall where the round arch outer side transitions into the round arch inner side, on both sides and increase the wall thickness of the cannula wall at this location. This additionally makes it possible for the bending to not only be effected in the cannula centre, but also to be distributed over a larger region.

The above-described reinforcing elements may be embodied such that they protrude slightly beyond the outer circumference of the cannula wall in the reinforced region, wherein a protrusion in the range of 0.05 to 0.30 mm is particularly preferred, as a result of which the tendency to kink in the region of the cannula centre is further reduced, firstly owing to the greater material thickness in this region and secondly since the inner cannula can be better supported on the inner wall of the outer cannula by way of the protruding reinforcing element.

Preferably, these reinforcing elements extend over an area which has a greater extent in the longitudinal direction of the cannula than in a transverse direction, wherein the area particularly preferably tapers from the centre thereof in the distal direction and/or in the proximal direction, in order to provide as little resistance as possible when introducing a correspondingly reinforced inner cannula into the outer cannula. In embodiments with ring-like, clasp-like or spiral-like indentations in the cannula wall, the thicker cannula wall portions, which run between the indentations, of one or more adjacent indentations are connected to one another. Preferably, the reinforcing elements are located laterally, i.e. in the region of the transition between the inner side of the round arch portion and the outer side of the round arch portion.

In certain embodiments, one or more guide strips may also be provided in the cannula wall, said guide strips extending continuously or interrupted in certain portions on the outer wall of the inner cannula in the longitudinal direction from proximal to distal. In some embodiments, at least one such guide strip extends in the longitudinal direction along the outer side of the round arch portion and/or at least one such guide strip extends in the longitudinal direction along the inner side of the round arch portion and/or at least one such guide strip extends in the longitudinal direction along a transition between the inner side of the round arch portion and the outer side of the round arch portion.

Preferably, these guide strips have a width in the range of 0.5 to 2.0 mm and protrude slightly beyond the outer circumference of the cannula wall, wherein a protrusion in the range of 0.05 to 0.30 mm is particularly preferred, as a result of which the introduction of the inner cannula into the outer cannula is facilitated.

When selecting the plastics material used for the inner cannula, fundamentally any material that provides the required stability and flexibility and that is tolerable to patients is suitable. In certain embodiments, the flexible plastics material of the cannula wall is selected from among polyurethane, polypropylene, polyethylene, polyethylene terephthalate, polyvinyl chloride, SEBS (polystyrene-polyethene-polybutene-polystyrene), SBS (polystyrene-polybutadiene-polystyrene), SIS (polystyrene-polyisoprene-polystyrene), IR (polyisoprene), silicone or mixtures of these polymers, such as polypropylene/polystyrene-polyethene-polybutene-polystyrene. Typically, the plastics material used has a Shore A hardness of < 95 so that the required flexibility is ensured.

According to one aspect of the present invention, the inner cannula proposed according to the invention is combined with an outer cannula to form a tracheostomy tube by virtue of the inner cannula according to the invention being arranged in the outer cannula. The tracheostomy tube according to the invention is produced by virtue of the inner cannula according to the invention being introduced into the outer cannula of a conventional tracheostomy tube. Accordingly, the inner cannula according to the invention may also be used for the exchange of a used inner cannula in a tracheostomy tube that comprises an outer cannula and an inner cannula arranged in the outer cannula.

In certain embodiments, a connection for connection to a mechanical ventilation device is located at the proximal end of the outer cannula of the tracheostomy tube. In an alternative embodiment, the connection for connection to a mechanical ventilation device is located at the proximal end of the inner cannula.

For the purposes of the original disclosure, it is pointed out that all the features which a person skilled in the art can gather from the present description, the appended drawings and the claims, even if they have been described specifically only in connection with certain further features, can be combined both individually and in any desired combinations with other features or groups of features disclosed herein, provided that this has not been expressly ruled out or such combinations are rendered impossible or pointless for technical reasons. Here, for the sake of brevity and readability of the description, a comprehensive, explicit presentation of all conceivable combinations of features is omitted.

Individual specific embodiments of the invention are schematically illustrated in the appended figures and the associated description of the figures. These specific embodiments are merely an example of possible combinations of features. The invention is, however, by no means limited to these specific embodiments, but rather encompasses all the embodiments covered by the scope of protection of the patent claims, even if they are not explicitly illustrated or described.
- Figure 1:: Figure 1 illustrates a longitudinal cross section through a schematic illustration of a conventional inner cannula.
- Figure 2:: Figure 2 shows, respectively, a cross section through the longitudinal axis of a highly schematically illustrated conventional inner cannula (left) and of a highly schematically illustrated inner cannula according to the invention (right).
- Figure 3:: Figure 3 illustrates a plan view of a schematic illustration of one embodiment of an inner cannula according to the invention.
- Figure 4:: Figure 4 illustrates a longitudinal cross section through a schematic illustration of one embodiment of an inner cannula according to the invention.
- Figure 5:: Figure 5 illustrates a plan view of a schematic illustration of a specific embodiment of an inner cannula according to the invention.

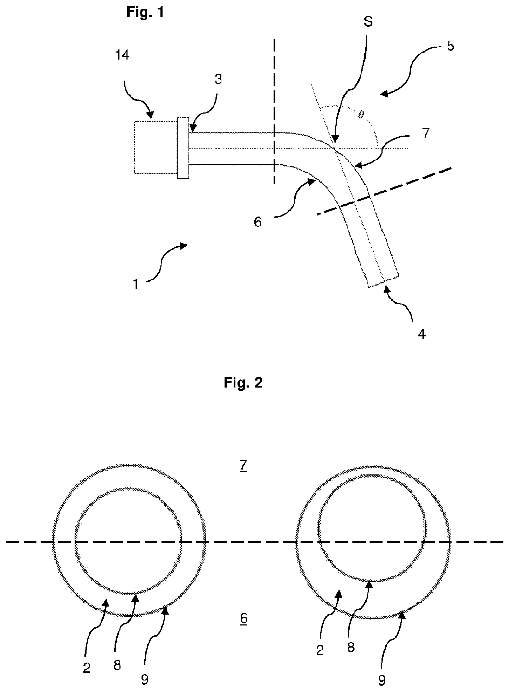

Figure 1 illustrates a longitudinal cross section through a schematic illustration of a conventional inner cannula 1 which extends in the longitudinal direction from the proximal end 3 thereof to the distal end 4 thereof, wherein a connection 14 for connection to a ventilation appliance is provided at the proximal end 3. A rectilinear longitudinal portion is located at the proximal end 3, just like at the distal end 4. Located in the region between the two dashed lines is the curved round arch portion 5 with an inner arcuate side 6 and an outer arcuate side 7. On the outer arcuate side 7, a longitudinal portion running on the outer arcuate side 7 is located between the two dashed lines, whilst, on the inner arcuate side 6, a longitudinal portion running on the inner arcuate side extends between the two dashed lines. The apex of the round arch on the outer arcuate side 7 is located at the location labelled with the letter S.

The left side of Figure 2 illustrates a cross section through a round arch portion of a conventional inner cannula. Here, the tubular cannula wall 2 is defined by a circular inner circumference 8 and a circular outer circumference 9. Illustrated on the right side is one embodiment of an inner cannula according to the invention in which the cannula wall 2 is also defined by a circular outer circumference 9 and a circular inner circumference 8, wherein here, unlike in the conventional design illustrated on the left side, the two circles of the outer circumference and the inner circumference are arranged eccentrically rather than concentrically, i.e. the circle centre of the inner circumference 8 is arranged offset in relation to the circle centre of the outer circumference 9. According to the invention, this eccentric arrangement is provided at least in certain portions in the round arch portion of the inner cannula and, in the embodiment illustrated here, is designed in such a way that the circle centre of the inner circumference is arranged offset in the direction of the outer arcuate side 7 of the round arch portion 5 such that the average wall thickness of the cannula wall on the inner arcuate site 6 is considerably greater than the average wall thickness on the opposite outer arcuate side 7.

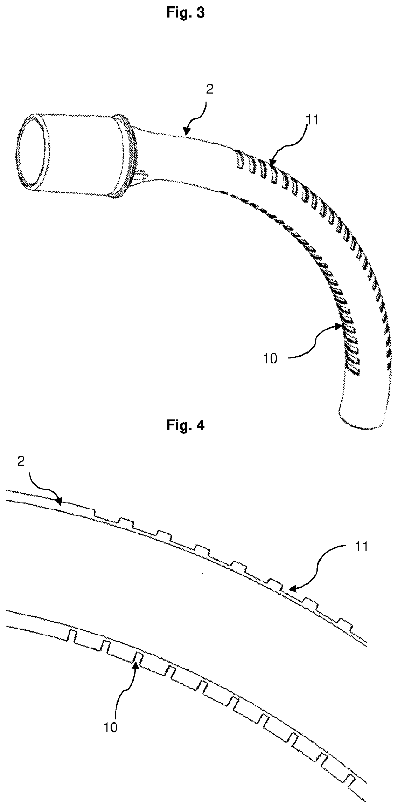

Figure 3 illustrates a plan view of a schematic illustration of one embodiment of an inner cannula according to the invention in which the thicker embodiment of the inner arcuate side in comparison to the outer arcuate side is effected by an eccentric arrangement of the circular inner circumference in relation to the circular outer circumference, as schematically illustrated in the illustration on the right side of Figure 2. In the embodiment illustrated here, indentations 11 are provided on the outer arcuate side. Furthermore, indentations 10 are also provided on the inner arcuate side.

Figure 4 illustrates a longitudinal cross section through a schematic illustration of one embodiment of an inner cannula according to the invention. In the embodiment illustrated here, the cannula wall on the outer arcuate side is reduced in terms of its average wall thickness in comparison with the cannula wall on the inner arcuate side by virtue of provision being made of indentations which are considerably wider than the indentations are deep. By contrast, indentations 10 which are of considerably deeper design in comparison to the width of the indentations are provided on the inner arcuate side. Due to the relatively wide indentations on the outer arcuate side, the outer arcuate side achieves a higher flexibility than the inner arcuate side. Due to the comparatively narrow indentations of greater depth on the inner arcuate side, the flanks of the wall portions between which the indentations lie can come to lie against one another in the case of a more pronounced curvature of the inner cannula, which results in a considerably higher kink resistance.

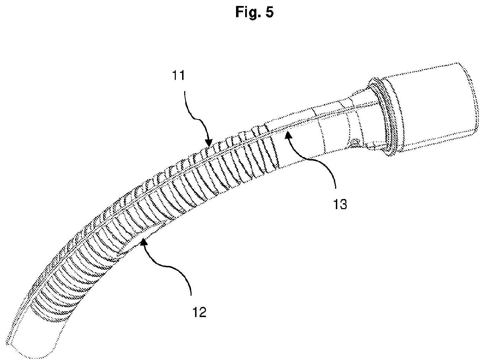

Figure 5 illustrates a plan view of a schematic illustration of a specific embodiment of an inner cannula according to the invention in which, as has already been schematically illustrated in Figure 4, the indentations 11 on the outer arcuate side are of relatively wide embodiment. However, at the transition to the inner arcuate side, these indentations are narrower and at the same time deeper, which also corresponds to the schematic illustration of the indentations on the inner arcuate side of Figure 4. In the embodiment illustrated here, as additional reinforcing means, a guide strip 13 is provided at the outermost circumference of the outer arcuate side and lateral reinforcing elements 12, of which only one is visible in the illustration of Figure 5, are provided on both sides at the transition of the round arch outer side to the round arch inner side.

### List of reference designations:

- 1: Inner cannula
- 2: Cannula wall
- 3: Proximal end
- 4: Distal end
- 5: Round arch portion
- 6: Inner arcuate side
- 7: Outer arcuate side
- 8: Inner circumference
- 9: Outer circumference
- 10: Indentation on the inner arcuate side
- 11: Indentation on the outer arcuate side
- 12: Reinforcing element
- 13: Guide strip
- 14: Connection
- S: Apex

## Claims

1. Inner cannula (1) for insertion into an outer cannula of a tracheostomy tube, wherein the inner cannula (1) comprises a tubular cannula wall (2) which is composed of flexible plastics material and which extends in a longitudinal direction between a proximal end (3) of the inner cannula (1) and an opposite distal end (4) of the inner cannula (1), wherein the inner cannula (1) comprises, in the longitudinal direction thereof, a round arch portion (5) with an inner arcuate side (6) and an outer arcuate side (7), wherein the average wall thickness of the cannula wall (2) at least along a cannula wall longitudinal portion running on the inner arcuate side (6) is at least 40% greater than the average wall thickness of the cannula wall (2) along a longitudinal portion running on the outer arcuate side (7) and lying opposite the longitudinal portion running on the inner arcuate side (6),
wherein the cannula wall (2) comprises indentations (10) in the cannula wall (2) at least in the region of the longitudinal portion running on the inner arcuate side (6) and indentations (11) in the cannula wall (2) at least in the region of the longitudinal portion running on the outer arcuate side (7),
wherein on the inner arcuate side (6), the maximum depth (T) of the indentations (10) is greater than the maximum width (B) thereof, and in that, on the outer arcuate side (7), the maximum depth of the indentations (11) is lower than the maximum width thereof.

2. Inner cannula (1) according to Claim 1, wherein at least in the region of the longitudinal portion running on the inner arcuate side (6) and of the longitudinal portion running on the outer arcuate side (7), the inner circumference (8) and the outer circumference (9) of the cannula wall (2) are each circular, elliptical or oval in cross section perpendicular to the central longitudinal axis of the inner cannula and are arranged such that the average wall thickness of the longitudinal portion on the inner arcuate side is at least 40% greater than the average wall thickness of the longitudinal portion on the outer arcuate side (7).

3. Inner cannula according to either of Claims 1 and 2, wherein on the inner arcuate side (6), the maximum depth of the indentations (10) is 10% to 70% greater than the maximum width thereof.

4. Inner cannula according to either of Claims 1-3, wherein on the outer arcuate side (7), the maximum depth of the indentations (11) is 10% to 70% lower than the maximum width thereof.

5. Inner cannula according to one of Claims 1 to 4, wherein the indentations (11) in the cannula wall (2) extend continuously or interrupted in certain portions in a ring-like, clasp-like or spiral-like manner in a circumferential direction.

6. Inner cannula according to one of Claims 1 to 5, wherein the flexible plastics material of the cannula wall is selected from among polyurethane, polypropylene, polyethylene, polyethylene terephthalate, polyvinyl chloride, SEBS (polystyrene-polyethene-polybutene-polystyrene), SBS (polystyrene-polybutadiene-polystyrene), SIS (polystyrene-polyisoprene-polystyrene), IR (polyisoprene), silicone or mixtures of these polymers, such as polypropylene/polystyrene-polyethene-polybutene-polystyrene.

7. Inner cannula according to one of Claims 1 to 6, wherein the flexible plastics material of the cannula wall has a Shore A hardness of <95.

8. Tracheostomy tube with an outer cannula and an inner cannula arranged in the outer cannula, wherein the inner cannula is an inner cannula according to one of Claims 1 to 7.

## Patentansprüche

1. Innenkanüle (1) zum Einschieben in eine Außenkanüle eines Tracheotomietubus, wobei die Innenkanüle (1) eine rohrförmige Kanülenwand (2) aus flexiblem Kunststoffmaterial umfasst, die sich zwischen einem proximalen Ende (3) der Innenkanüle (1) und einem gegenüberliegenden distalen Ende (4) der Innenkanüle (1) in Längsrichtung erstreckt, wobei die Innenkanüle (1) in deren Längsrichtung einen Rundbogenabschnitt (5) mit einer Innenbogenseite (6) und einer Außenbogenseite (7) umfasst, wobei wenigstens entlang eines auf der Innenbogenseite (6) verlaufenden Längsabschnittes der Kanülenwand die mittlere Wandstärke der Kanülenwand (2) wenigstens 40% größer ist als die mittlere Wandstärke der Kanülenwand (2) entlang eines auf der Außenbogenseite (7) verlaufenden Längsabschnittes, der dem auf der Innenbogenseite (6) verlaufenden Längsabschnittes gegenüberliegt, wobei die Kanülenwand (2) wenigstens in dem Bereich des auf der Innenbogenseite (6) verlaufenden Längsabschnittes Vertiefungen (10) in der Kanülenwand (2) umfasst und wenigstens in dem Bereich des auf der Außenbogenseite (7) verlaufenden Längsabschnittes Vertiefungen (11) in der Kanülenwand (2) umfasst,
wobei auf der Innenbogenseite (6) die maximale Tiefe (T) der Vertiefungen (10) größer ist als deren maximale Breite (B) und auf der Außenbogenseite (7) die maximale Tiefe der Vertiefungen (11) geringer ist als deren maximale Breite.

2. Innenkanüle (1) nach Anspruch 1, wobei wenigstens in dem Bereich des auf der Innenbogenseite (6) verlaufenden Längsabschnittes und des auf der Außenbogenseite (7) verlaufenden Längsabschnittes der Innenumfang (8) und der Außenumfang (9) der Kanülenwand (2) im Querschnitt senkrecht zur zentralen Längsachse der Innenkanüle jeweils kreisförmig, elliptisch oder oval sind und so angeordnet sind, dass die mittlere Wandstärke des Längsabschnittes auf der Innenbogenseite wenigstens 40% größer ist als die mittlere Wandstärke des Längsabschnittes auf der Außenbogenseite (7).

3. Innenkanüle nach einem der Ansprüche 1 und 2, wobei auf der Innenbogenseite (6) die maximale Tiefe der Vertiefungen (10) 10% bis 70% größer ist als deren maximale Breite.

4. Innenkanüle nach einem der Ansprüche 1-3, wobei auf der Außenbogenseite (7) die maximale Tiefe der Vertiefungen (11) 10% bis 70% geringer ist als deren maximale Breite.

5. Innenkanüle nach einem der Ansprüche 1 bis 4, wobei sich die Vertiefungen (11) in der Kanülenwand (2) durchgehend oder abschnittsweise unterbrochen in Umfangsrichtung ringförmig, spangenförmig oder spiralförmig erstrecken.

6. Innenkanüle nach einem der Ansprüche 1 bis 5, wobei das flexible Kunststoffmaterial der Kanülenwand ausgewählt ist unter Polyurethan, Polypropylen, Polyethylen, Polyethylenterephthalat, Polyvinylchlorid, SEBS (Polystyrol-Polyethen-Polybuten-Polystyrol), SBS (Polystyrol-Polybutadien-Polystyroltyrol), SIS (Polystyrol-Polyisopren-Polystyrol), IR (Polyisopren), Silikon oder Mischungen dieser Polymere, wie Polypropylen/ Polystyrol-Polyethen-Polybuten-Polystyrol.

7. Innenkanüle nach einem der Ansprüche 1 bis 6, wobei das flexible Kunststoffmaterial der Kanülenwand eine Shore A Härte von <95 aufweist.

8. Tracheotomietubus mit einer Außenkanüle und einer in der Außenkanüle angeordneten Innenkanüle, wobei die Innenkanüle eine Innenkanüle nach einem der Ansprüche 1 bis 7 ist.

## Revendications

1. Canule interne (1) destinée à être insérée dans une canule externe d'un tube de trachéotomie, la canule interne (1) comprenant une paroi de canule tubulaire (2) qui est composée de matière plastique souple et qui s'étend dans une direction longitudinale entre une extrémité proximale (3) de la canule interne (1) et une extrémité distale opposée (4) de la canule interne (1), la canule interne (1) comprenant, dans sa direction longitudinale, une partie arquée ronde (5) dotée d'un côté arqué interne (6) et d'un côté arqué externe (7), l'épaisseur de paroi moyenne de la paroi de canule (2) au moins le long d'une partie longitudinale de paroi de canule s'étendant sur le côté arqué interne (6) étant supérieure d'au moins 40 % à l'épaisseur de paroi moyenne de la paroi de canule (2) le long d'une partie longitudinale s'étendant sur le côté arqué externe (7) et située à l'opposé de la partie longitudinale s'étendant sur le côté arqué interne (6), la paroi de canule (2) comprenant des indentations (10) dans la paroi de canule (2) au moins dans la région de la partie longitudinale s'étendant sur le côté arqué interne (6) et des indentations (11) dans la paroi de canule (2) au moins dans la région de la partie longitudinale s'étendant sur le côté arqué externe (7),
dans laquelle sur le côté arqué interne (6), la profondeur maximale (T) des indentations (10) est supérieure à la largeur maximale (B) de celles-ci, et en ce que, sur le côté arqué externe (7), la profondeur maximale des indentations (11) est inférieure à la largeur maximale de celles-ci.

2. Canule interne (1) selon la revendication 1, dans laquelle au moins dans la région de la partie longitudinale s'étendant sur le côté arqué interne (6) et de la partie longitudinale s'étendant sur le côté arqué externe (7), la circonférence interne (8) et la circonférence externe (9) de la paroi de canule (2) présentent chacune une section transversale circulaire, elliptique ou ovale perpendiculaire à l'axe longitudinal central de la canule interne et sont agencées de telle sorte que l'épaisseur de paroi moyenne de la partie longitudinale sur le côté arqué interne soit supérieure d'au moins 40 % à l'épaisseur de paroi moyenne de la partie longitudinale sur le côté arqué externe (7).

3. Canule interne selon l'une ou l'autre des revendications 1 et 2, dans laquelle sur le côté interne arqué (6), la profondeur maximale des indentations (10) est supérieure de 10 % à 70 % à la largeur maximale de celles-ci.

4. Canule interne selon l'une ou l'autre des revendications 1 à 3, dans laquelle sur le côté arqué externe (7), la profondeur maximale des indentations (11) est inférieure de 10 % à 70 % à la largeur maximale de celles-ci.

5. Canule interne selon l'une des revendications 1 à 4, dans laquelle les indentations (11) dans la paroi de canule (2) s'étendent de manière continue ou interrompue dans certaines parties de façon annulaire, en forme de pince ou en spirale dans la direction circonférentielle.

6. Canule interne selon l'une des revendications 1 à 5, dans laquelle la matière plastique souple de la paroi de canule est choisie parmi polyuréthane, polypropylène, polyéthylène, polyéthylène téréphtalate , chlorure de polyvinyle, SEBS (polystyrène-polyéthène-polybutène-polystyrène), SBS (polystyrène-polybutadiène-polystyrène), SIS (polystyrène-polyisoprène-polystyrène), IR (polyisoprène), silicone ou des mélanges de ces polymères, tels que polypropylène/polystyrène-polyéthène-polybutène-polystyrène.

7. Canule interne selon l'une des revendications 1 à 6, dans laquelle la matière plastique souple de la paroi de canule présente une dureté Shore A <95.

8. Tube de trachéotomie doté d'une canule externe et d'une canule interne agencée dans la canule externe, dans lequel la canule interne est une canule interne selon l'une des revendications 1 à 7.
